# EUROPEAN PATENT APPLICATION

(11) **EP 1 767 921 A1**
(43) Date of publication of application: **28.03.2007**
(21) Application number: 05077189.8
(22) Date of filing: 26.09.2005
(51) Int. Cl.: G01N 3/08, G01N 3/32, G01N 19/02

(54) **Method for determining the suitability of a material as infill material in artificial turf systems**

(71) Applicant: DSM IP Assets B.V., 6411 TE Heerlen (NL)
(72) Inventor: Bruls, Wilhelmus Gerardus Marie, 6243 BE Geulle (NL); Linsen, Jozef Maria Herman, 6431 TM Hoensbroek (NL); Steeman, Paulus Antonius Maria, 6176 JC Spaubeek (NL); Op den Buijsch, François Antoine Marie, 6367 HN Voerendaal (NL)

(57) **Abstract**

Method for determining the suitability of a material as infill material in artificial turf fields, comprising the steps of subjecting a sample of the material to a combination of a static force and a dynamic force superimposed on said static force, said dynamic force being imposed with a frequency chosen in the range of 0.1 to 100 Hz, measuring at least the dynamic force and the corresponding impression of the sample and deriving relevant parameters from the measured data and from the sample after having been subjected to said forces.

## Description

The invention relates to a method for determining the suitability of a material as infill material in artificial turf fields.

Artificial turf fields for playing soccer and other ball games are becoming more and more popular. The two main components of such fields are artificial grass fibers and infill material, spread out between the fibers up to a certain part of their height. Other components that may be present are e.g. sand for anchoring the field to the ground. The whole is laid upon a functional underground, that also may contribute to the total performance of the field.

The infill material usually is a resilient material, e.g. rubber granules. Its function is to damp the shocks that ankles and knees of the players may suffer when they run and jump on the field. Further the infill material must have sufficient elasticity so as to give the playing ball bouncing characteristics similar to a natural grass field.

The artificial field must retain these properties for an acceptable period of time since removing and replacing the infill material is a costly and cumbersome task. As an illustration, a soccer field has an area of about 10,000 square meters and the infill layer has a thickness of typically 2 - 2.5 cm, resulting in some 200 to 250 m³ of infill material to be removed from between the grass fibers when the infill material is to be replaced.

The international football association FIFA has imposed certain test criteria an artificial turf field has to comply with. These criteria comprise values that mainly depend on the infill material, e.g. for the shock absorption, vertical impression, energy restitution, vertical rebound and further criteria that are mainly associated with the artificial grass fibers applied. In addition also the physical sensation, the 'feel', that players experience is tested in practice. Materials possessing the required combination of properties have been found to show visco-elastic properties, i.e. part of the energy fed to it, e.g. by a running person or a bouncing ball should be given back but also part of it must be absorbed.

In present practice the properties of infill material are determined on test fields specifically laid out for this purpose and using equipment specifically designed for this purpose. Fields, the properties of which do not comply with the required criteria have to be cleared away, which involves serious efforts and a waste of material. An even greater problem lies in the longer-term behavior of the infill material. It has appeared that infill material that initially passes the FIFA test deteriorates in a relatively short time to an unacceptable level causing the infill material to have to be removed and replaced over the full area of a playing field.

Thus, there is a need for a method for determining the suitability of a material as infill material in artificial turf fields, that can be used to select infill material that complies with the official requirements and that provides sufficient information to predict the longer-term behavior of the tested material.

The invention now provides such a method in that it is subjecting a sample of the material to a combination of a static force and a dynamic force superimposed on said static force, said dynamic force being imposed with a frequency chosen in the range of 0.01 to 100 Hz, measuring at least the dynamic force and the corresponding impression of the sample and deriving relevant parameters from the measured data and from the sample after having been subjected to said forces.

It has been found that deriving a number of relevant properties on just a small sample, e.g. less than 1 kg or even less than 500 g or even less than 100 g of the material, can predict the suitability and the longer-term behavior of the material. Also a natural feel for the players, e.g. response on running and jumping comparable to that of a natural grass field, was found to be associated with the materials thus selected.

The method according to the invention has as a further advantage that only the properties of the infill material are measured, whereas in the known on-the-field methods properties of the combination of grass fibers, sand and infill material are determined. In the latter situation it is more difficult to predict the behavior of the infill material as such. The test method according to the invention can also be applied to a sample of a field, containing all the above-mentioned components.

In the method according to the invention a sample of the material is subjected to a combination of a static force and a dynamic force. The static force, which is kept constant, imposes a certain impression on the sample.
The amount of impression depends on the force applied and the visco-elastic properties of the material. On this static force a dynamic force is superimposed. The resulting total force thus lies between the static force plus and minus the amplitude of the dynamic force. The dynamic force is imposed with a certain frequency, generally between 0.01 and 100 Hz. Its waveform may be deliberately chosen but a sinusoidal form is very suitable. The described combination of forces can be applied with techniques known per se for this, e.g. with Dynamic Mechanical Analysis (DMA). Instruments for applying this technique are known per se and are commercially available.

With this instrument the combination of the momentarily imposed force and the corresponding impression is measured and usually recorded for further evaluation, e.g. via Fourier analysis. Combining these data with the geometry of the testing equipment (such as a contact area on which the force is applied), and the thickness of the sample allows characteristics of the sample to be calculated in a manner known per se.

At this point it is pointed out that there is a relation between the imposed force and the stress on the test sample, this stress being the force divided by the area on which the force is exerted. The DMA apparatus normally applies a force. The value and amplitude of the force to be applied can be calculated from the desired stress to be put on the sample and the area of the stamp that is used to impose the force.

An analogous relationship exists between the resulting impression and the strain, the strain being the impression divided by the thickness of the sample. Thus, the force and the impression are absolute values, expressed in N(ewton) and mm, whereas the stress and the strain are the resulting sample size independent values, expressed in Pa(scal) and %. In the following the terms force and stresses or impression and strain may be used alternatively, the relation being clear from the above explanation.

It is further noted that it is both possible to impose a defined stress (converted to a required force) and measure the resulting impression (that can be converted to the resulting strain) or to impose a defined impression (calculated from a desired strain) and measure the required force (that can be converted to the corresponding stress). In the following the procedure will be disclosed starting from a defined force, causing a resulting impression but it will be clear for the skilled person how to derive the reverse situation from that disclosure.

When a periodical force with a certain frequency is imposed on a visco-elastic material, it has appeared that the corresponding impression shows a phase shift, i.e. a shift in time, with respect to the imposed force. This phase shift or phase angle is commonly denoted as δ. Another parameter measured in DMA techniques is the dynamic modulus E_{d}, that can be derived from the stress-strain data as quotient of the stress (derived from the force) and strain (derived from the impression) amplitudes. This dynamic modulus can be resolved into the elastic or storage modulus E' (=E_{d}*cosδ) and the viscous or loss modulus E" (=E_{d}*sinδ). The quotient E"/E' is equal to tan δ, the loss tangent, whereas E_{d}=√(E'²+E"²). All these parameters depend on the frequency and the temperature at which the measurements are performed. Standard DMA equipment records the force-impression data, calculates the stress-strain data, and converts these to the dynamic modulus and phase angle online.

Preferably the dynamic force is imposed on the sample with a number, e.g. a least 3, e.g. 8 steps per decade, of frequencies in the defined frequency range. Advantageously these frequencies are, evenly on a logarithmic scale, spread over the range. In this way the method more accurately reflects the behavior of the infill material under the various type of stresses it will experience in practice. E.g., the force exerted by a running person has a different frequency pattern than a bouncing ball. The number of steps per decade is chosen so as to locate the frequency range in which the response to the forces is smooth and gradually, i.e. without peaks or upswings due to experimental artifacts. Evaluation of the data then can be based on the values obtained at one point within said frequency range. A frequency of 1 Hz has been found to be very suitable in most cases. Another advantage of applying the forces over a broad range of frequencies will become apparent later in this description when a further decisive parameter, the flow retention, is discussed.

For more representative results the static load is preferably removed between consecutive measurements with different frequencies.

The static force that imposes a constant impression is chosen so as to represent the mean load exerted by a running person. Static forces, generating static stresses up to about 500 kPa, may be applied for this purpose. In general, static forces generating a stress of 10 to 80 kPa have been found representative. Preferably static forces causing a stress of between 15 and 40 kPa are applied since this range is considered to be representative for the typical feet pressure during walking and running.

Smaller and larger forces can be used but preferably the static force is chosen corresponding to the specified pressure ranges.

The amplitude of the dynamic force is usually chosen within the same range as the static force but preferably lower than the static force, preferably between 10 and 90 %, more preferably between 10 and 75% of the static force.

If the test is carried in the reverse way, i.e. by imposing a defined static and dynamic impression and measuring the required forces, a static impression corresponding to a strain of 5 - 30 % was found suitable. The superimposed dynamic impression is as for the force, so usually chosen within the same range as the static impression but preferably lower than the static impression, preferably between 10 and 90 %, more preferably between 10 and 75% of the static impression.

The sample material is tested like it will be applied as infill material, i.e. in the same granular or powdery form. The sample preferably is placed in a sample holder that limits movement of the sample in directions perpendicular to the direction of the imposed forces. This prevents the sample from escaping from under the object,
e.g. a stamp plate, exerting the force, which in fact would reduce the amount of sample subjected to the forces. This requirement is technically simply met with a flat-bottomed cup having an outer edge and one or more walls rising perpendicularly from preferably the whole of said outer edge. Thus a cup is obtained having a continuous sidewall in which the sample is enclosed when a flat stamp is applied to the top of the sample through the open side of the cup opposite to the bottom. The continuous sidewall then prevents the sample from escaping from under the stamp. The sidewall is preferably cylindrical but can also be multi-angular and in that case it is preferably rectangular and more preferably square.

The area of the open side of the cup determines the area of sample tested and is preferably at least 10 cm² and more preferably at least 20 cm². Lower values may cause side effects from the vicinity of the solid walls. From the viewpoint of the method applied there is in fact no limitation to the measuring area but from a practical viewpoint, e.g. considering the maximum force that can be imposed by the used instrument, the dimensions of that instrument and the amount of sample required, said area is advantageously chosen below 200 cm².

The thickness of the sample can also vary between broad limits but in order to minimize or even eliminate the influence of the, solid, bottom this thickness preferably is at least 5 mm, more preferably at least 10 mm and most preferably at least 15 mm. The maximum thickness is not critical but as a practical upper limit 100 or 50 mm can be chosen. Values around the usual thickness of the infill layers in turf fields of 20 - 25 mm will be quite satisfactory.

A stamp imposes the forces on the sample. This stamp preferably is flat to impose an even force on the sample over its whole area. The area and shape of the stamp may be chosen deliberately as long as it fits within the opening of the cup. A foot shaped stamp may be applied. Preferably, however, its shape matches that of the opening of the cup and also preferably it fits slidingly within this opening, i.e. with a minimal space between the outer edge of the stamp and the inside of the opening, just preventing the stamp and the inside of the cup from being into contact in order to avoid friction and loss of force. However, a small clearance between stamp and cup wall is allowable, preferably of less than 10 % of the radius or largest diagonal of the cup, in order to prevent the sample from escaping from under the stamp through the opening between the outer edge of the stamp and the wall of the cup. This clearance preferably is smaller than the granule diameter of the sample to be tested.

A further relevant parameter, apart from those that can be derived from the stress-strain data, and critical for the prediction of the longer time behavior of the tested infill material, was found to be the flow retention. The flow retention is defined here as the percentage of the mass of the sample particles (powder, granules, pellets or other form) that flows individually from the sample holder after completion of the test program when the sample holder is tilted over 180 ° with respect to the (normally horizontal) test position. Flow retention of 75% or more has been found representative for a material showing acceptable to excellent longer-term behavior.

Since this flow retention may be dependent on the number of times the force has been applied and the frequency and magnitude of the force applied, it is recommended to adopt the procedure described above regarding the frequency range and the number of steps per decade. In addition it is preferred to perform the measurements at consecutive frequencies (preferably from low to high) such that first the static force is equilibrated for at least ten seconds, followed by dynamic measurements during at least ten cycles, and unloading between the measurement frequencies. From the DMA measurements thus performed the dynamic modulus E_{d} and the loss tangent (tan δ) are obtained as a function of the frequency. After inspecting that the curves are indeed smooth as discussed before, the data at a frequency of 1 Hz are selected for further evaluation. Having obtained the values for tan δ and the dynamic modulus, the material can be assessed for its suitability as infill material. For a material to be suitable for this purpose the vertical impression and the energy absorption must lie in the ranges prescribed by the football association standards as a minimum requirement. It was found that the values of the dynamic modulus and the loss tangent obtained with the test method according to this invention can be used as indicators for this performance. A low value of the dynamic modulus will result in too much impression, i.e. a very soft feeling of the field, while a high value will result in too low impression, i.e. a very stiff feeling of the field. Similarly, a low value of the loss tangent will result in too little energy absorption while a high value of the loss tangent will result in too much energy absorption. Consequently, optimal ranges for both the dynamic modulus and the loss tangent can be defined to mimic the actual requirements set by the football association.

In the method according to the invention the optimum ranges for the dynamic modulus and the phase angle, at a measurement frequency of 1Hz and room temperature (23°C), were found to be:
0.5 MPa ≤ dynamic modulus E_{d} ≤ 2.0 MPa and
0.19 ≤ tan δ ≤ 0.36

Preferably the lower limit of the dynamic modulus is at least 0.65 MPa and more preferably at least 0.8 MPa. Preferably the upper limit of the dynamic modulus is at most 1.6 MPa and more preferably at most 1.3 MPa. Preferably the lower limit of the tan δ is at least 0.22 and more preferably at least 0.25. Preferably the upper limit of the tan δ is at most 0.34 and more preferably at most 0.32

However, these values are not limiting to the applicability of the measurement technique according to this invention but just reflect the selection criteria found to match with the present requirements for artificial turf fields.

The flow retention as defined below must be at least acceptable but preferably is excellent according to the criteria given below.

The invention will be elucidated by the following examples, without being restricted thereto.

In the examples a GABO Eplexor 500N dynamic spectrometer was used with a load cell of 500N.

### Experimental set-up and Experiment I

In the GABO Eplexor 500N spectrometer as the sample holder a cylindrical cup having an internal diameter of 50 mm and having a cylindrical sidewall of 20 mm internal height was used. The load arm was provided with a cylindrical compression plate having a diameter of 48 mm, thus fitting within the inner diameter of the cup. The cup was filled with 15 g of a sample material (sample 1 in Table 1) to be tested. The material is known to meet the requirements set by the football association. Prior to the test the sample was loaded and unloaded three times with a static force of 10 Newton to obtain stable impression. The measurements for the actual frequency sweep were carried out at a temperature of T=23±2°C. Measurements were carried out at 8 frequencies equally spaced on a logarithmic scale within a frequency range of 0.1-100 Hz.

As a first step a static impression was applied in order to obtain a static force of 50 Newtons with a tolerance of ±1N. The static force was measured at 1 s intervals, and the static impression adapted automatically, until ten consecutive measurements show a stable static force of 50±1 N. To avoid instrument damage, the maximum static deformation is limited to 60% of the original sample height. Subsequently a sinusoidal dynamic impression was applied in order to superimpose a dynamic force with an amplitude of 25 N (tolerance ±1 N) on the static force. Stress and strain were measured, and the amplitude of the dynamic impression adapted automatically, until 10 cycles with a dynamic force of 25±1 N have been obtained. To avoid instrument damage, the maximum dynamic deformation was limited to 40% of the sample thickness. From these ten cycles the dynamic modulus and tan delta are evaluated. The results for sample 1 are listed in Table 1.

Using these measurement conditions the applied static stress equals about 27 kPa, and the applied dynamic stress amplitude about 14 kPa, which is within the defined optimum ranges.

### Examples II - VIII

In the same equipment as in Example I 15 grams of various materials proposed or contemplated as infill material was placed in the cup. The measurement conditions were identical to Example I. The materials used (samples 2-9) and the test results are shown in Table 1.

**Table 1**

| Sample | Material | E_{d}(MPa) | Tan δ | Compacting |
|---|---|---|---|---|
| 1 | ATH filled plastomer (ground) | 1.1 | 0.29 | ++ |
| 2 | ATH filled plastomer (micro granulate) | 4.1 | 0.12 | ++ |
| 3 | Linear low density polyethylene (plant powder) | 10.0 | 0.33 | + |
| 4 | Linear low density polyethylene (granulated and ground) | 3.9 | 0.41 | ++ |
| 5 | ATH filled linear low density polyethylene (granulated and ground) | 6.5 | 0.39 | ++ |
| 6 | Thermoplastic elastomer | 0.94 | 0.17 | ++ |
| 7 | Crosslinked EPDM rubber 1 (ground) | 1.4 | 0.33 | - |
| 8 | Crosslinked EPDM rubber 2 (ground) | 1.1 | 0.28 | - |
| 9 | Crosslinked EPDM rubber 2 one year old | 1.8 | 0.29 | - |

| | | | | |
|---|---|---|---|---|
| Compacting: ++ (Excellent) Virtually all granules/powder flow individually from the sample holder + (Reasonable) 75-95% of the granules/powder flow individually from the sample holder +/- (Insufficient) 25-75% of the granules/powder flow individually from sample holder, remainder sticks together as a cake - (Bad) More than 75% of the sample sticks together as a cake | | | | |

From these results it follows that materials 2, 3, 4, 5 and 6 do not have the required dynamic modulus and/or tan δ value and thus only for this reason are not suitable as infill material. Further it appears that materials 7 and 8 although meeting the requirement for modulus and tan δ, after the test are so compacted that they do not flow as the original powder or granules from the sample holder but to a large extent or even completely have been formed into a compact cake. For this reason these materials, at least in the tested form, do not qualify as suitable infill material.

### Example IX

A sample was taken from a material that has been used as infill material on a soccer field for about 1 year. The sample consisted of individually ground granules. The playing characteristics after that year had become unsatisfactory. Before applying it as infill material on the field the material had been tested on a test field and judged as satisfactorily then.

The sample was tested as described in Example I (results see sample 9 in Table 1). The same material in the form as originally applied as infill material was also tested (results see sample 8 in Table 1).

It appeared that the original material satisfies the requirement for dynamic modulus and tan δ but after the test formed a compacted cake that did not fall out of the sample holder when this was held upside down. The sample taken from the one year old field appeared to have a much higher dynamic modulus than the original material and did not fall out of the sample holder when this was held upside down, so its compacting properties were still bad. From this it can be concluded that this material, although found in the beginning satisfactory in field tests, from the onset would not have past a selection based on the method of the present invention.

## Claims

1. Method for determining the suitability of a material as infill material in artificial turf fields, comprising the steps of subjecting a sample of the material to a combination of a static force and a dynamic force superimposed on said static force, said dynamic force being imposed with a frequency chosen in the range of 0.1 to 100 Hz, measuring at least the dynamic force and the corresponding impression of the sample and deriving relevant parameters from the measured data and from the sample after having been subjected to said forces.

2. Method according to claim 1, wherein as relevant parameters at least the dynamic modulus, the tangent of the phase angle and the flow retention are measured.

3. Method according to claim 1 or 2, wherein the dynamic force on the sample is imposed consecutively with a number of frequencies within said range.

4. Method according to any one of claims 1 to 3, wherein the static force is chosen within the range imposing a static stress on the sample from 10 to 80 kPa.

5. Method according to any one of claims 1 to 4, wherein the dynamic force is chosen within the range from 10 to 90 % of the static force.

6. Method according to any one of claims 1 to 5, wherein the sample is placed in a sample holder that limits movement of the sample in directions perpendicular to the direction of the dynamic force.

7. Method according to claim 6, wherein the sample holder is a cylindrical or multi-angular cup having a flat bottom having an outer edge and one or more walls raising from the whole of said outer edge.

8. Method according to claim 6 or 7, wherein the area of the cup is between 20 cm² and 200 cm².

9. Method according to any one of claims 1 to 8, wherein the sample has a weight of less than 100 g.

10. Method according to any one of claims 6 to 9, wherein the forces are imposed by a flat plate having the same shape as the sample holder and fitting within the cup slidingly or with a clearance of at most 10% of a radius or a largest diagonal of the cup.

11. Method according to any one of claims 6 to 10, wherein the sample beforeimposing the forces forms a layer in the cup with a height of between 5 and 50 mm.

12. Method according to any one of claims 6 to 11, wherein the ratio between the height of the sample and the square root of the area is in the range of 0.5 to 10.

13. Method according to any one of claims 1 to 12, further comprising the step of selecting a material having at 1Hz and room temperature as tan δ a value of between 0.19 and 0.36 and as dynamic modulus E_{d} a value between 0.5 MPa and 2.0 MPa and excellent flow retention.
